# EUROPEAN PATENT APPLICATION

(11) **EP 2 330 522 A1**
(43) Date of publication of application: **08.06.2011**
(21) Application number: 10014490.6
(22) Date of filing: 20.06.2002
(51) Int. Cl.: G06F 19/00

(54) **Method and system for integrated medical tracking**

(30) Priority: 20.06.2001 US 299629 P
(62) Divisional of application: 02742247.6
(71) Applicant: Tyco Healthcare Group LP, Mansfield, MA 02048 (US)
(72) Inventor: Whitman, Michael P., New Hope, PA 18938 (US)
(74) Representative: Maschio, Antonio

(57) **Abstract**

A system for processing data provided by medical devices. The system includes a medical device having a memory device configured to store medical device information. The system also includes an information system connected to the medical device. The information system includes at least a server configured to receive at least a portion of the medical device information from the memory device of the medical device. The server is also configured to employ the medical device information to process at least one patient data, prescription data and inventory/ordering data. The medical device information may include serial number data, device identifier data, operation data and/or usage date for the medical device. The patient data may include at least one of patient tracking data, patient recordkeeping data and patient billing data. The prescription data may include prescription issuance data, prescription filling data and prescription tracking data. The inventory/ordering data may include at least one of maintenance and replacement schedule data, administrator notification data, and automatically-generated medical device order data.

## Description

### CROSS-REFERENCE TO RELATED APPLICATIONS

The present application claims the benefit of U.S. Patent Application Serial No. 60/299,629, filed on June 20, 2001, which is expressly incorporated herein by reference in its entirety.

This patent application is related to U.S. Patent Application Serial No. 09/836,781, filed on April 17, 2001 entitled "Electro-Mechanical Surgical Device" and U.S. Patent Application Serial No. 09/887,789 filed June 22, 2001 entitled "Electro-Mechanical Surgical Device", each of which is incorporated in its entirety by reference herein.

### FIELD OF THE INVENTION

The present invention relates to a method and system for tracking medical information. In particular, the present invention relates to the tracking of information provided by medical devices including electro-mechanical surgical devices.

### BACKGROUND INFORMATION

Innovation in the medical device arena is an ongoing process taking advantage of general technological and scientific trends and developments. Medical devices may now include memory devices that store information that may be used to assist in medical procedures. For example, information relating to the medical device and/or attachment may also be stored such as, *inter alia,* the serial number of the device/attachment and a device/attachment identifier. In addition to device/attachment specific information, usage related data (e.g., the number of times a device/attachment has been used) may also be stored in newer generations of medical devices and, in particular, with surgical devices.

In one example, a circular stapler surgical attachment may be used in conjunction with an electro-mechanical surgical device. The electro-mechanical surgical device may read information related to the circular stapler attachment such as the serial number of the attachment and an attachment identifier indicating a circular stapler. One purpose for the collection of this information may be to determine whether a surgical device and/or attachment has been used up to or beyond a threshold limit requiring replacement or maintenance of the device and/or attachment. In the case the threshold is reached, the electro-mechanical surgical device may generate an audio and/or video message indicating the condition and may not function until properly replaced and/or maintained. U.S. Patent Application Serial No. 09/836,781, filed on April 17, 2001 entitled "Electro-Mechanical Surgical Device", and U.S. Patent Application Serial No. 09/887,789 filed June 22, 2001 entitled "Electro-Mechanical Surgical Device", each of which is expressly incorporated herein in its entirety by reference, discuss and reference other examples of medical devices (e.g., electro-mechanical surgical devices) that collect and store such information. Another purpose for the collection of such information may be to determine which one of a number of algorithms should be used to drive the surgical device and/or attachment, based on, for example, the attachment identifier.

The management of hospitals and other medical care providers is undergoing innovation and evolution similar to the technical advances being made in medical devices. These management innovations are particularly relevant in the field of information systems. Hospital and/or medical care information systems track, for example, patient information, prescriptions, inventory, and ordering data. There are multiple benefits from these systems. For example, patient information systems provide more accurate patient tracking, record keeping, and billing. Prescription information systems automate the prescription issuing, filling, and tracking process providing greater control over and accuracy in the medicine dispensed. Inventory and ordering information systems automate and facilitate the ordering of new medical inventory and provide comprehensive order tracking and analysis. Though various hospital and medical information systems have improved medical care management, limited integration of systems and data have prevented greater benefits from being realized.

### SUMMARY

In accordance with example embodiments of the present invention, a method and system for integrating the data tracked by medical devices with hospital and medical information systems is provided. The present invention solves a long-felt need for more accurate medical tracking by automatically collecting and integrating medical device data with hospital-wide and/or other medical information systems in order to provide more thorough, integrated, and accurate data collection.

In one example embodiment, the present invention links one or more medical devices (e.g., electro-mechanical surgical devices) with a hospital information system to link medical device data with other hospital data such as, for example, patient and billing information. The integration of data in this exemplary embodiment may allow detailed accounting of which devices were used in treating a patient, at what time, and to what degree. This information may be retained as part of a patient's file and may be used for billing and/or insurance purposes. Additionally, the integrated tracking of this data may allow a hospital and/or other medical care provider to more accurately determine and/or project its equipment maintenance and replacement needs. The integration of medical device data with a hospital information system may further allow automated inventory tracking and ordering whereby a threshold stock of an medical device, attachment, and/or other item may be automatically maintained.

In an alternative embodiment, the present invention links one or more medical devices (e.g., an electro-mechanical surgical device) with an integrated information system serving as a repository of medical device data. The integrated information system may in turn be linked to other hospital and/or medical information systems thereby allowing the medical device data to be integrated with other data such as, for example, patient data as discussed above. This alternative embodiment differs from the exemplary embodiment in that it implements one or more separate medical device data repositories and/or information systems which in turn are linked with other hospital and/or medical information systems rather than directly integrating the medical device data with the hospital and/or medical information systems.

One aspect of the present invention is the manner in which a medical device is linked to an information system. In the exemplary embodiment of the present invention, a medical device may incorporate a processor, memory, and a data storage device. Medical device data may be stored locally by the medical device and then transmitted at some interval or in real-time or near real-time for integration with one or more other hospital and/or medical information systems. In an alternative embodiment regarding this aspect of the present invention, medical device data may be directly transmitted and not locally stored.

### BRIEF DESCRIPTION OF THE DRAWINGS

FIG. 1 is an illustration of one embodiment of a medical device―in this instance an electro-mechanical surgical device―that electronically tracks information and that may, according to one embodiment of the present invention be linked to a hospital and/or medical information system.
FIG. 2 is a schematic depiction of an exemplary electro-mechanical device and connection arrangement to connect the medical device with a server and/or an information system according to one embodiment of the present invention.
FIG. 3 is a block diagram depicting an example of a network topology illustrating the relationship between the server, a storage device, and the medical devices placed in various rooms of a hospital according to one embodiment of the present invention.
FIG. 4 is a block diagram depicting an example of a network topology depicting the interconnection between a first medical device and a server over an information network without using an intermediary second medical device according to one embodiment of the present invention.
FIG. 5 is a block diagram depicting an example of a network topology depicting the interconnection between a medical device, a hospitable and/or medical information system, and an automated ordering system according to one embodiment of the present invention.

### DETAILED DESCRIPTION

In accordance with an example embodiment of the present invention, a method and system for the automatic integration of medical device data with one or more hospital and/or medical information systems is provided. The present invention relates to medical devices including surgical devices and attachments that electronically collect and/or determine information. For example, an electro-mechanical surgical device such as the devices disclosed in U.S. Patent Application Serial No. 09/723,715, filed on November 28, 2000, entitled "Electro-Mechanical Surgical Device" and U.S. Patent Application Serial No. 09/836,781, filed on April 17, 2001 entitled "Electro-Mechanical Surgical Device", and U.S. Patent Application Serial No. 09/887,789 filed June 22, 2001 entitled "Electro-Mechanical Surgical Device", each of which are expressly incorporated herein in its entirety, are several such medical devices. A practitioner skilled in the art will recognize that this patent application refers to any such medical device and therefore this patent application should not be construed as limiting the present invention to only the device disclosed in the aforementioned patent application. Other medical devices may include surgical and non-surgical medical instruments and attachments.

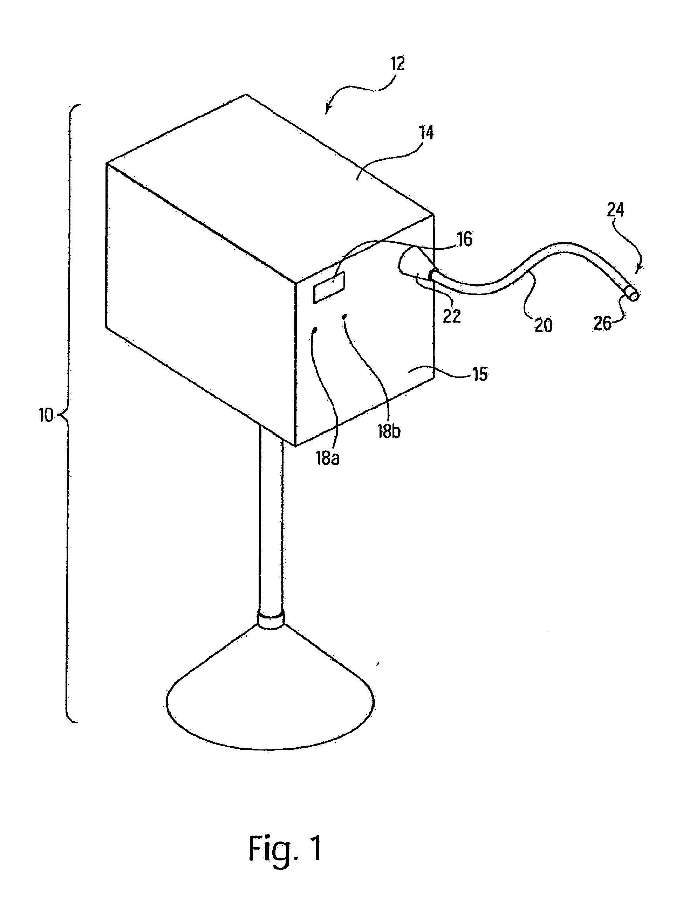

FIG. 1 is an illustration of one embodiment of a medical device - in this instance an electro-mechanical surgical device ― that electronically tracks information and that may, according to one embodiment of the present invention be linked to a hospital and/or medical information system. The exemplary medical device 10 may include, for example, a remote power console 12, which includes a housing 14, having a front panel 15 on which are mounted a display device 16 and indicators 18a, 18b. A flexible shaft 20 may extend from the housing 14 and may be detachably connected via a first coupling 22. The distal end 24 of the flexible shaft 20 may include a second coupling 26 adapted to detachably secure a surgical instrument and/or attachment to said distal end 24 of the flexible shaft 20. The surgical instrument or attachment may be, for example, a surgical stapler, a surgical cutter, a surgical stapler-cutter, a linear surgical stapler, a linear surgical stapler-cutter, a circular surgical stapler, a circular surgical stapler-cutter, a surgical clip applier, a surgical clip ligator, a surgical clamping device, a vessel expanding device, a lumen expanding device, a scalpel, a fluid delivery device or any other type of medical instrument. Some of such medical instruments are described, for example, in U.S. Patent Application Serial No. 09/324,451 which issued as U.S. Patent No. 6,315,184, entitled "A Stapling Device for Use with an Electromechanical Driver Device for Use with Anastomosing, Stapling, and Resecting Instruments," U.S. Patent Application Serial No. 09/324,452, entitled "Electromechanical Driver Device for Use with Anastomosing, Stapling, and Resecting Instruments," U.S. Patent Application Serial No. 09/351,534 which issued as U.S. Patent No. 6,264,087, entitled "Automated Surgical Stapling System," U.S. Patent Application Serial No. 09/510, 926 which issued as U.S. Patent No. 6,348,061, entitled "A Vessel and Lumen Expander Attachment for Use with an Electromechanical Driver Device," U.S. Patent Application Serial No. 09/510,927, entitled "Electromechanical Driver and Remote Surgical Instruments Attachment Having Computer Assisted Control Capabilities," U.S. Patent Application Serial No. 09/510,931, entitled "A Tissue Stapling Attachment for Use with an Electromechanical Driver Device," U.S. Patent Application Serial No. 09/510,932, entitled "A Fluid Deliver Mechanism for Use with Anastomosing, Stapling, and Resecting Instruments," U.S. Patent Application Serial No. 09/510,933, entitled "A Fluid Delivery Device for Use with Anastomosing, Stapling, and Resecting Instruments," U.S. Patent Application Serial No. 09/09/887,789, entitled "Surgical Device with Intracorporally Attachable Instrument," U.S. Patent Application Serial No. 09/873,682, entitled "Parallel Jaw Device For Use With an Electro-Mechanical Driver Device," U.S. Patent Application Serial No. 09/999,546, entitled "Linear Stapler/Cutter Device," U.S. Patent Application Serial No. 09/990,067, entitled "A Vessel and Lumen Expander Attachment For Use With an Electro-Mechanical Driver Device," U.S. Patent Application Serial No. 10/094,051, entitled "Right Angle Linear Surgical Cutter" and U.S. Patent Application Serial No. 10/099,634, entitled "Flexible Drive Shaft for Coupling a Surgical Attachment to an Electro-Mechanical Driver Device," each of which is expressly incorporated in its entirety herein by reference.

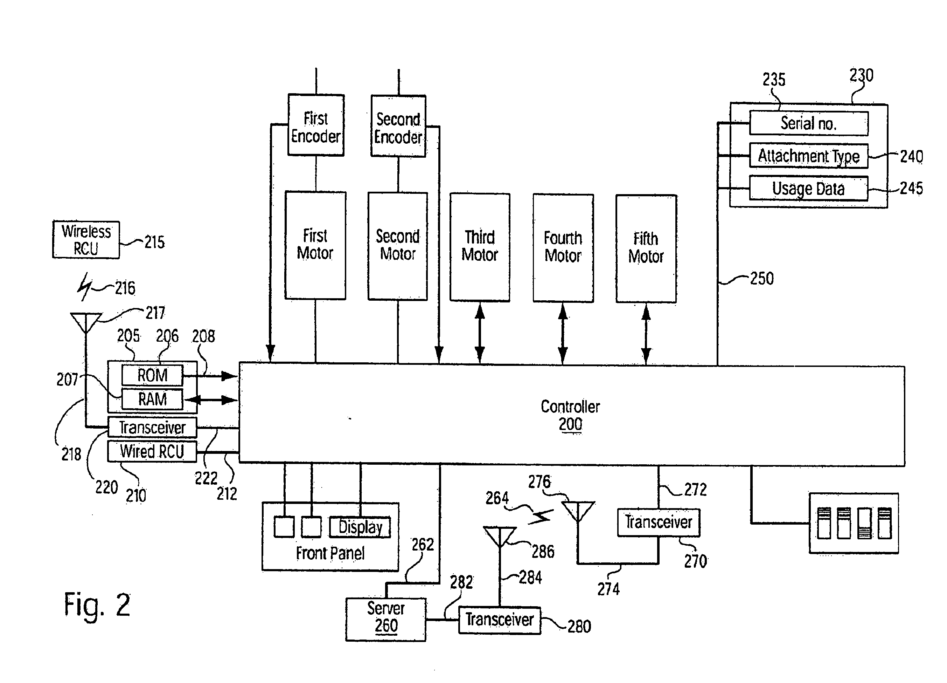

FIG. 2 is a schematic depiction of an exemplary electro-mechanical device and connection arrangement to connect the medical device with a server and/or an information system according to one embodiment of the present invention. A controller 200 is provided in the housing 14 of a remote power console 12 and is configured to control functions and operations of the electro-mechanical surgical device 10 and any surgical instrument or attachment attached to the flexible shaft 20. A memory unit 205 is provided and may include memory devices, such as, a ROM component 206 and/or a RAM component 207. ROM component 206 is in electrical and logical communication with controller 200 via line 208. RAM component 207 may include any type of random-access memory, such as, for example, a magnetic memory device, an optical memory device, a magneto-optical memory device, an electronic memory device, etc. Similarly, ROM component 206 may include any type of read-only memory, such as, for example, a removable memory device, such as a PC-Card or PCMCIA-type device. ROM component 206 and RAM component 207 may be embodied as a single unit or may be separate units. A wired remote control unit ("RCU) 210 may be electrically and logically connected to the controller 200 via a line 212. A wireless RCU 215 may be provided and may communicate via a wireless link 216 with a receiving/sending unit 217 connected via line 218 to a transceiver 220. The transceiver 220 may be electrically and logically connected to the controller 200 via line 222.

A memory unit 230 may be contained within the surgical instrument and/or attachment attached to the electro-mechanical surgical device described herein as an example of a medical device for use in accordance with the example embodiment of the present invention. The memory unit 230 of the instrument or attachment may be configured to store, for example, serial number data 235, attachment type identifier ("ID") data 240, and usage data 245. The memory unit 230 may additionally store other and/or alternative data. The memory unit 230 of a surgical instrument and/or attachment may be electrically and logically connected to the controller 200 via, for example, a line 250 of a data transfer cable enclosed in the flexible shaft 20 of the remote console 10. Because the memory unit of a surgical instrument and/or attachment may store collected data directly, it may, in one embodiment of the present invention, serve as a medical device directly connectable with a server as described below without the intermediate connection with a remote console 10.

In accordance with an example embodiment of the present invention, the medical device may include a wired and/or wireless connection between the medical device and a server and/or an information system. As depicted in FIG. 2, a wired connection between the remote console 10 and a server 260 may exist over a communications line 262. A medical device may also be connected to a server using a wireless connection 264. For example, the medical device may include, *inter alia,* a wireless transmitter or transceiver 270 that can communicate with a receiver or transceiver 280 at the server 260. The transmitter or transceiver 270 on the medical device end of the wireless connection 264 may be electrically and/or logically connected to the controller 200 via a line 272. The receiver or transceiver 280 on the server 260 end of the wireless connection 264 may be electrically and/or logically connected to the server 260 via a line 282. The wireless link 264 may be, for example, a radio link, an optical link, such as an infrared link, or any other form of wireless communication link.

In the example embodiment, a medical care provider (e.g., a hospital) may use a number of medical devices, such as the remote console discussed above. The medical devices may be distributed across an entire medical care facility. For example, these medical devices may be located in several different rooms of a hospital.

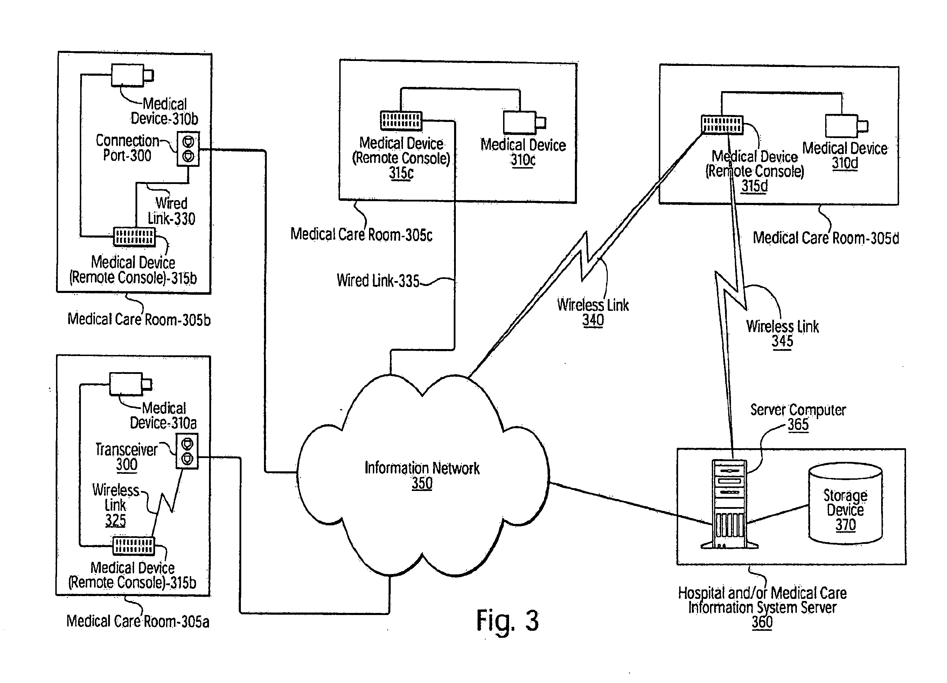

FIG. 3 is a block diagram depicting an example of a network topology illustrating the relationship between the server, a storage device, and the medical devices placed in various rooms of a hospital according to one embodiment of the present invention. According to the exemplary embodiment of the present invention, one or more medical devices 310a-310d, 315a-315d may be located in a hospital or other medical care provider facility. These medical devices 310a-310d, 315a-315d may be mobile (i.e., easily moveable) or they may be affixed (e.g., attached to a wall or floor in the room). Mobile medical devices may be placed in various rooms 305a-305d within the medical facility and may be moved to other rooms 305a-305d as needed while affixed medical devices may be dedicated for use in a particular room 305a-305d. The medical rooms 305a-305d may serve as general treatment or diagnoses rooms or may be assigned to a particular specialty, such as obstetrics/gynecology (ob/gyn), urology, general surgery, colon/rectal, etc. The medical devices 310a-310d, 315a-315d may be connected to a server 360 by various connection mechanisms as discussed below.

A server 360 may be one or more devices containing at least one processor (not shown but part of 365), memory (not shown but part of 365), and possibly a storage device 370. Typically, a server 360 will be one or more computer systems 365 that may include a variety of processing and storage devices 370. According to the example embodiment of the present invention, a server 360 may already be part of an existing hospital and/or other care provider computer network 350 and may host all or part of a hospital and/or other medical information system (not shown). For example, an already existing hospital server 360 may contain patient data that may be accessed by terminals or client computer devices distributed throughout the hospital. The patient data contained or accessed by the server 360 may be part of a patient management or billing information system (not shown). A server communicates with a client device (e.g., a medical device) 315a-315d over an information network 350. Both servers 360 and clients 315a-315d, would therefore include, for example, appropriate network connection devices such as, for example, Ethernet network interface cards or built in Ethernet capability. The information network 350 over which the server communicates with the client may be any information network such as, *inter alia,* the Internet, public switched telephone network ("PSTN"), local area network ("LAN"), wide area network ("WAN"), metropolitan area network ("MAN"), and wireless network.

In the exemplary embodiment illustrated in FIG. 3, a first medical device 310a-310d, e.g., a surgical and/or medical attachment, is connected to a second medical device 315a-315d, e.g., a remote console and/or control unit. The first medical device 310a-310d may store data such as, for example, a serial number and a device type. The first medical device 310a-310d may also store usage information such as, for example, an indicator of whether or not the device has ever been used and/or the number of times the device has been used (e.g., a counter). The second medical device 315a-315d may then read some or all of the stored information and may, in turn, generate and/or store additional data such as date and time stamps, an indication of the length of time the first and/or second medical device was operated during a given procedure, an indication of the department that used the device, an indication of the treatment room within which the device was used, an serial number of the second medical device, usage information regarding the second medical device, etc. The second medical device 315a-315d, as shown in one embodiment of the present invention depicted in FIG. 3, may then transmit the medical data to a server 360 over the information network 350.

The medical device 315a-315d may be connected to an information network 350 via a number of communication arrangements such as, for example, a hard-wired connection 335, a detachable wired connection 330, and a wireless connection 325, 340. A hard-wired connection 335 has a cable or other connection directly into a medical device 315c where the communication line is not easily detachable hence the "hard-wired" nature of the connection. A hard-wired connection 335 though prevalent with earlier network connected devices is less common today. A detachable wired connection 330 also has a cable or other connection directly to a medical device 315b except this cable connection is easily detachable usually with the cable or other wire connection plugging into a port on the medical device. The cable or other wire connection may also be connected to a connection port 300 in the room 305b with this connection port 300 typically being hard-wired into the information network 350. A wireless connection 325, 340 has, at both ends of the wireless connection, a transceiver or other device that can transmit and/or receive a wireless signal. A medical device 315a may have a transceiver (not shown) embedded in the device and may implement a wireless link 325 to another transceiver 320 in the medical care room 305a. This medical care room 305a transceiver 320 may be hard-wired into the information network 350. A medical device 315d may also implement a wireless link 340 to the information network 350 with an information network 350 containing a transceiver (not shown) located outside the medical care room 305d. A medical device 315d may also bypass the information network 350 and directly implement a wireless link 345 with a computer 365 and associated transceiver (not shown) of the hospital and/or medical care information system server 360. Regardless of the implementation of the wireless connection, the wireless link 325, 340, 345 and signal maybe optical (e.g., infrared), a radio link, or any other form of wireless communication.

A first medical device 310a-310d such as a surgical instrument and/or attachment that would otherwise in the exemplary embodiment be connected to a second medical device 315a-315d such as a remote console may directly connect to the server 360 according to an alternative embodiment of the present invention. This connection as previously stated may be hardwired, wired, and/or wireless. The most advantageous of these connections may be a wireless connection because of the additional flexibility available to the medical practitioner (e.g., the doctor, nurse, internist, etc.) using the first medical device 310a-310d.

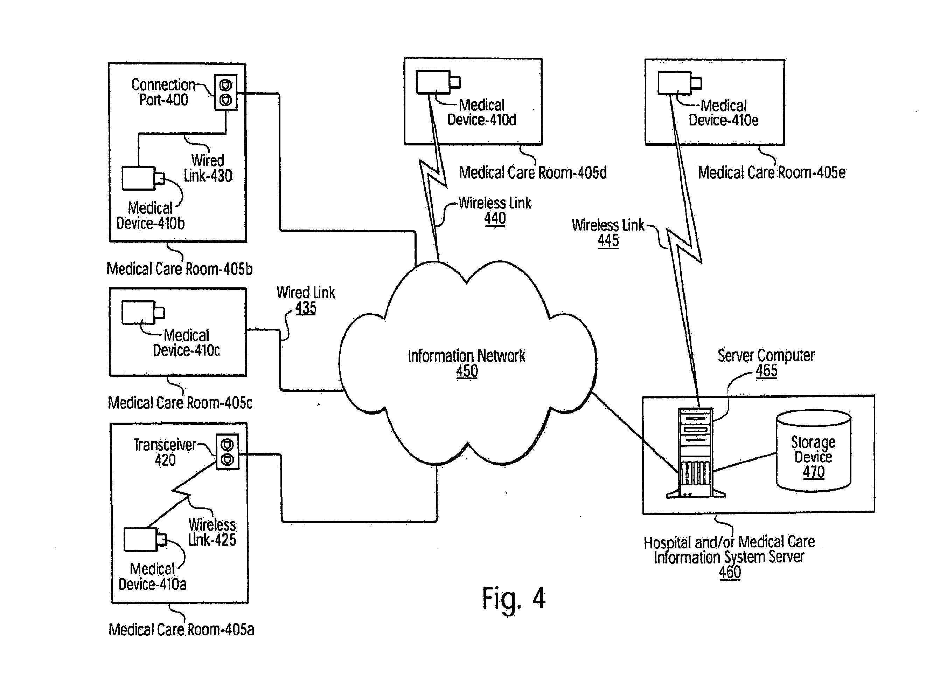

FIG. 4 is a block diagram depicting an example of a network topology showing the interconnection between a first medical device and a server over an information network without using an intermediary second medical device according to one embodiment of the present invention. According to this alternative embodiment of the present invention, one or more medical devices 410a-410e may be located in various medical care rooms 405a-405e of a hospital or other medical care facility. The medical devices 410a-410e may be mobile or affixed to the medical care rooms 405a-405e in which they are located. The medical devices 410a-410e may be connected to a hospital and/or medical care information system server 460 over an information network 450. The information system server 460 may include, for example, a server computer 465 containing a processor (not shown), memory (not shown), and one or more storage devices 470. The information system may host all or part of a hospital and/or medical care information system (i.e., software and data that are not shown).

The example embodiment of the present invention shown in FIG. 4 illustrates a first medical device 410a-410e, such as a surgical instrument and/or attachment, directly connected to a server 460 over an information network 450 without using a second, intermediary medical device such as a remote console. The means by which the medical devices 410a-410e connect to the information network 450 and server 460 are similar to means the second medical devices 315a-315d were connected as discussed with FIG. 3. A medical device 410c may have a hardwired connection 435 to the information network 450 directly from a medical care room 405c. A medical device 410b may also have a detachable wired connection 430 where a cable or other wire may detachably be connected to the medical device 410b and may detachably be connected with some connection port 400 located in a medical care room 405b. The connection port 400 may be hardwired to the information network 450. A medical device 410a, 410d-410e may also have a wireless connection 425, 440, 450 with an information network 450 or server 460. A medical device 410a may have an embedded transceiver (not shown) to send and receive wireless signals. The medical device 410a may communicate over a wireless link 425 with a transceiver 420 located in a medical care room 405a. This transceiver 420 may be hardwired to the information network 450. A medical device 410d with an embedded transceiver (not shown) may also communicate over a wireless link 440 wireless connection with another transceiver (not shown) connected to the information network 450 where the transceiver (not shown) is not located in the same medical care room 405d. A medical device 410e with a transceiver (not shown) may also implement a wireless connection 445 directly with a server 460 through a transceiver (not shown) connected to the a server computer 465.

In another embodiment of the present invention, a first medical device (e.g., a surgical instrument and/or attachment) may connect to both a second medical device (e.g., a remote console) and the server in a combination of the embodiments illustrated in FIGS. 3 and 4. For example, a first medical device (e.g., a surgical stapler) may connect to a remote console using a wired connection (i.e., a cable). The same first medical device may also connect directly to a server using a wireless communication link. This communication link may be implemented with a transceiver in the first medical device transmitting and/or receiving wireless signals with a second transceiver located either in the same medical care room, elsewhere on the information network, or at the server.

The data collected and/or determined by a medical device may include, for example, a serial number of the medical device, a device and/or attachment type identifier data, and/or usage data according to one embodiment of the present invention. In other embodiments of the present invention, other types of data may also and/or alternatively be collected and/or determined by a medical device. This data may be collected and/or determined by a first and/or second medical device and transmitted to a server as described above. The transfer of collected and/or determined medical data may be performed interactively in real-time according to the exemplary embodiment of the present invention. The real-time data transfer may involve the transmission of data as it is collected and/or determined. In an alternative embodiment of the present invention, the transfer of data may be performed periodically where at some interval data is transferred from a medical device to a server. For example, a medical device may be configured to transfer batch data at some predetermined, preprogrammed (e.g., upon reaching some threshold limit), and/or random interval.

The collected and/or determined medical data may be transferred to a central repository as indicated in FIGS. 3 and 4 according to one embodiment of the present invention. The central repository may be one or more databases wherein the data is stored. The one or more databases of the central repository may be distinct and separate from the databases used by other hospital and/or medical information systems in one embodiment of the present invention. This distinct integrated medical device database(s) ("integrated information system") may consolidate the medical data sent to the server by one or more various medical devices. According to this embodiment, the hospital and/or medical care information system server (i.e., the server) may also be distinct from the server(s) hosting other hospital and/or medical information systems. Additionally, the integrated information system, though a centralized repository, may use a distributed information system with, for example, parts of the repository spread over several servers according to one embodiment of the present invention.

In an alternative embodiment of the present invention, the medical device data may be integrated directly into the tables and/or files of another existing hospital and/or medical information system, such as a patient information system, rather than first being integrated into a separate integrated information system. According to this embodiment, medical device data may be directly integrated with one or more hospital information systems through a data transmission interface. This interface may be a software program or program element residing on a medical device, on the server, and/or elsewhere on the information network.

The collected and/or determined medical data may alternatively be transferred to an intermediate, decentralized repository, such as a departmental repository according to one embodiment of the present invention. According to this alternative embodiment, departments or groups of departments in a hospital and/or other medical care provider may have a separate server and integrated information system. Medical device data for devices belonging to this department or group of departments is sent to the respective decentralized (i.e., departmental) integrated information system. Information from these departmental integrated information systems may either be further integrated into a centralized integrated information system or may be directly integrated into other hospital and medical information systems.

The medical device data, whether first sent to a centralized integrated information system, a decentralized departmental integrated information system, or directly to a hospital and/or medical information system according to various embodiments of the present invention, may ultimately be integrated with a hospital and/or medical information system according to one embodiment of the present invention. For example, medical device data may be integrated with a patient information system whereby detailed information about the medical devices used and the particulars of the usage for a patient are stored and available with the patient's electronic record. This information may be used, for example, to more fully evaluate treatment, determine causes for medical complications, or for billing purposes.

In another example, medical device data may be integrated with a billing information system whereby detailed information about the medical device and its usage may be used in determining billing and may be included in a billing invoice. For example, a bill or invoice may include a specific breakdown of the equipment usage or treatment provided based wholly or in part on the medical device data provided. This information may also be sent to insurance providers for the patient and may be part of an insurance information system maintained by the hospital and/or other medical care provider. For example, the automated collection of medical device data may both assist insurance providers in obtaining more detailed information concerning patient treatment and may reduce the amount of time a hospital and/or other medical care provider needs to process information for insurance reporting.

The medical device data may also be used as part of an accounting information system to track, for example, medical device usage, revenues generated for the usage, and associated costs. For example, the medical device data may be used in a determination of the depreciation of the medical device based on an estimated number of times the medical device may be used before needing replacement. In this foregoing example, the medical device data is used to track medical device usage for only one possible financial and/or accounting purpose. Additionally, the medical device data may be used in determining financial information such as the revenue generated per usage or marginal revenue per usage of the medical device. The medical device data may also help a hospital and/or other medical care provider in determining the profitability of certain equipment and procedures and may assist in the more equitable pricing of such services. The medical device data may also be useful in calculating cost per usage, marginal cost per usage, and other cost related data.

Medical device data may also be integrated with an FDA tracking and/or monitoring information system whereby the information is used to provide the FDA information necessary for evaluating the efficacy of a device and/or procedure or for other statistical and/or reporting purposes.

Medical device data may also be integrated with an inventory control system whereby the medical device usage information is used to calculate and schedule maintenance and replacement of medical devices and thereby avoid complications by overusing medical devices. In this regard, medical device data may also be used to notify an administrator that a particular medical device (e.g., a disposable attachment) needs to be reordered when it drops below a particular level of stock. In an alternative embodiment, medical device data may also be used as part of an automatic ordering system where orders are automatically placed for a medical device (e.g., a disposable surgical attachment).

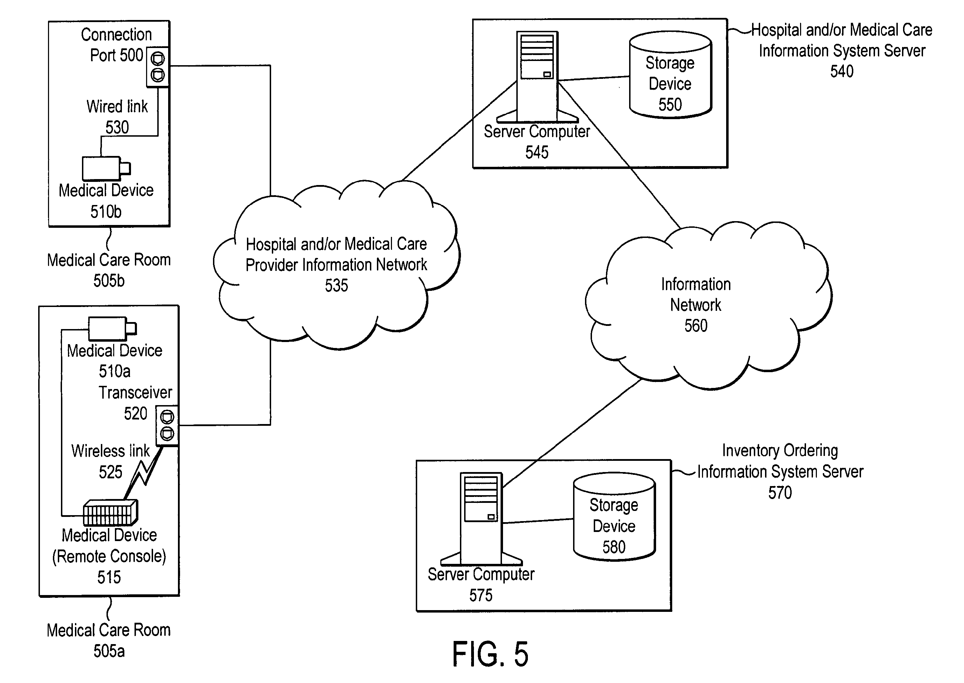

FIG. 5 is a block diagram depicting an example of a network topology depicting the interconnection between a medical device, a hospital and/or medical care information system and server, and an automated ordering system and server according to one embodiment of the present invention. FIG. 5 depicts one embodiment of the present invention in which at least one medical device 510a, 510b, 515 in a medical care room 505a, 505b of a medical care provider supplies data to an information system server 540 as previously discussed. In one medical care room 505a, a first medical device 510a may be connected to a second medical device 515 such as a remote console. The second medical device 515 may be connected via a wireless connection 525 to a transceiver 520 in the room 505a that may itself be connected to the hospital and/or medical care provider information network 535 with, for example, a hard-wired connection. In a second medical care room 505b, a first medical device 510b is connected to a hospital and/or medical care provider information network 535 by means of a detachable wired link 530. The wired link is detachably connected, in this example embodiment, to both the first medical device 505b and a connection port 500 in the room. The connection port 500 may be hard-wired to the medical care provider information network 535. The medical device data may be sent over the medical care information network 535 to the information system server 540 containing at least one of a computer 545 and a storage device 550. The data may be stored with one or more information systems on the storage device 550 of the server 540.

One hospital and/or medical care provider information system may include, in whole or in part, an inventory control system and/or order entry system according to one embodiment of the present invention. As mentioned above, the medical device data may be used to track the status and/or inventory of the particular medical device, such as, for example, a surgical attachment. When a new medical device needs to be replaced and/or on-hand inventory falls below some threshold limit, an order may be placed over an external information network 560, such as, for example, the Internet. The order may be placed with another server 570 containing a supplier inventory ordering system (not shown) that may be stored on a storage device 580 of the server 570 and run on the server's 570 computer 575.

The present invention may allow the entire process of inventory tracking to be greatly automated. This automation may extend from the automatic generation of medical device data and its automatic transmission to a medical provider information system to the automatic ordering of inventory when necessary. In one embodiment of the present invention, the automation of the inventory control and/or ordering process may eliminate or significantly reduce the need for human intervention in the process.

The present invention may also allow for a medical provider information system, such as the hospital and/or medical care information system server 540 to receive software via the information network 560, such as the Internet. For example, the operating software employed by the hospital and/or medical care information system server 540 to process the medical device data that it receives from the medical devices, such as medical devices 510a,510b, may be received by the hospital and/or medical care information system server 540 via Internet, or else the hospital and/or medical care information system server 540 may receives updated versions of such operating software via Internet. In this manner, the software that is employed by the hospital and/or medical care information system server 540 to process the medical device data that it receives from the medical devices may be periodically changed if desired.

The example hospital and/or other medical information systems discussed above with which medical device data may be integrated is merely exemplary. Actual hospital and/or other medical information systems may encompass many aspects of the several separately discussed examples above and may contain additional functions with which the integrated medical device data may be particularly advantageous. For example, a single hospital and/or medical information system may include patient, billing, and insurance information and reporting features. A practitioner skilled in the art will recognize that the medical device data may be automatically and electronically integrated with a wide variety of hospital and/or other medical information systems.

The invention may be described by reference to the following numbered paragraphs:
1. A system for processing data provided by a medical device, comprising a medical device including a memory device configured to store medical device information and an information system coupled to the medical device and configured to receive at least a portion of the medical device information, the information system further configured to process at least one of patient data, prescription data and inventory/ordering data as a function of at least a portion of the medical device information.
2. The system of paragraph 1, wherein the medical device information includes at least one of medical device serial number data, medical device identifier data, medical device operation data and medical device usage data.
3. The system of paragraph 1, wherein information system includes at least one of a processor, a memory device and a storage device.
4. The system of paragraph 1, wherein the information system is coupled to the medical device via an information network, the information network including at least one of the Internet, a public switched telephone network, a local area network, a wide area network, a metropolitan area network, and a wireless network.
5. The system of paragraph 4, wherein the information network is the Internet, and the information system is configured to receive updated software via the Internet.
6. The system of paragraph I, wherein the information system processes the patient data as a function of the at least portion of the medical device information, the patient data including at least one of patient tracking data, patient recordkeeping data and patient billing data.
7. The system of paragraph I, wherein the information system processes the prescription data as a function of the at least portion of the medical device data, and wherein prescription data includes prescription issuance data, prescription filling data and prescription tracking data.
8. The system of paragraph 1, wherein the information system processes the inventory/ordering data as a function of the at least portion of the medical device, and wherein the inventory/ordering data includes at least one of maintenance and replacement schedule data, administrator notification data, and automatically-generated medical device order data.
9. The system of paragraph 1, wherein the medical device is connected to the information system via a wired data connection.
10. The system of paragraph 1, wherein the medical device is connected to the information system via a wireless data connection.
11. The system of paragraph 1, wherein the medical device includes an electro-mechanical driver device, and a surgical device coupled to the electro-mechanical driver device.
12. The system of paragraph 11, wherein the memory device is positioned within the surgical device.
13. The system of paragraph 12, wherein the medical device is configured to at least one of store, generate and process data corresponding to at least one of a date the medical device is operated, a time the medical device is operated, a length of time that the medical device is operated, a medical facility department that used the medical device, a medical facility room in which the medical device is used, a serial number of the second medical device and a usage of the second medical device.
14. The system of paragraph 1, wherein the information system, wherein the intermediate information system includes a medical facility information network connected to a medical facility server, and wherein the medical facility server is configured to process at least one of patient data, prescription data and inventory/ordering data.
15. The system of paragraph 1, wherein the medical device is one of a surgical stapler, a surgical cutter, a surgical stapler-cutter, a linear surgical stapler, a linear surgical staplercutter, a circular surgical stapler, a circular surgical stapler-cutter, a surgical clip applier, a surgical clip ligator, a surgical clamping device, a vessel expanding device, a lumen expanding device, a scalpel and a fluid delivery device.
16. A method for processing data provided by a medical device, comprising receiving by an information system at least a portion of medical device information from a memory device of a medical device, processing at least one of patient data, prescription data and inventory/ordering data as a function of at least a portion of the medical device information.
17. The method of paragraph 16, wherein the receiving step includes receiving at least one of medical device serial number data, medical device identifier data, medical device operation data and medical device usage data, from the memory device.
18. The method of paragraph 16, further comprising connecting the medical device to the information system via an information network, the information network including at least one of the Internet, a public switched telephone network, a local area network, a wide area network, a metropolitan area network, and a wireless network.
19. The method of paragraph 18, wherein the connecting step includes connecting the medical devices to the information system via the Internet, the method further comprising receiving by the medical device updated software via the Internet.
20. The method of paragraph 16, wherein the processing step includes processing patient data that includes at least one of patient tracking data, patient recordkeeping data and patient billing data.
21. The method of paragraph 16, wherein the processing step includes processing prescription data that includes prescription issuance data, prescription filling data and prescription tracking data.
22. The method of paragraph 16, wherein the processing step includes processing inventory/ordering data that includes at least one of maintenance and replacement schedule data, administrator notification data, and automatically-generated medical device order data.
23. The method of paragraph 18, wherein the connecting step includes connecting the medical device to the information system via a wired data connection.
24. The method of paragraph 18, wherein the connecting step includes connecting the medical device to the information system via a wireless data connection.
25. The method of paragraph 16, wherein the medical device includes an electromechanical driver device and a surgical attachment coupled to the electro-mechanical device.
26. The method of paragraph 16, further comprising at least one of storing, generating and processing data, by the medical device, the data corresponding to at least one of a date the medical device is operated, a time the medical device is operated, a length of time that the medical device is operated, a medical facility department that used the medical device, a medical facility room in which the medical device is used, a serial number of the second medical device and a usage of the second medical device.

## Claims

1. A system for processing data provided by a medical device, comprising:
a medical device including a memory device configured to store medical device information; and
an information system coupled to the medical device and configured to receive at least a portion of the medical device information, the information system further configured to store at least one of patient data, prescription data and inventory/ordering data and process the at least one of patient data, prescription data and inventory/ordering data as a function of at least a portion of the medical device information.

2. The system according to claim 1, wherein the medical device information includes at least one of medical device serial number data, medical device identifier data, medical device operation data and medical device usage data.

3. The system according to any of the preceding claims, wherein the information system includes at least one of a processor, a memory device and a storage device.

4. The system according to any of the preceding claims, wherein the information system is coupled to the medical device via an information network, the information network including at least one of the Internet, a public switched telephone network, a local area network, a wide area network, a metropolitan area network, and a wireless network.

5. The system according to any of the preceding claims, wherein the information network is the Internet, and the information system is configured to receive updated software via the Internet.

6. The system according to any of the preceding claims, wherein the information system processes the patient data as a function of the at least portion of the medical device information, the patient data including at least one of patient tracking data, patient recordkeeping data and patient billing data.

7. The system according to any of the preceding claims, wherein the information system processes the prescription data as a function of the at least portion of the medical device data, and wherein prescription data includes prescription issuance data, prescription filling data and prescription tracking data.

8. The system according to any of the preceding claims, wherein the information system processes the inventory/ordering data as a function of the at least portion of the medical device, and wherein the inventory/ordering data includes at least one of maintenance and replacement schedule data, administrator notification data, and automatically-generated medical device order data.

9. The system according to any of the preceding claims, wherein the medical device is connected to the information system via a wired data connection.

10. The system according to any of the preceding claims, wherein the medical device is connected to the information system via a wireless data connection.

11. The system according to any of the preceding claims, wherein the medical device includes an electro-mechanical driver device, and a surgical device coupled to the electro-mechanical driver device.

12. The system according to any of the preceding claims, wherein the memory device is positioned within the surgical device.

13. The system according to claim 12, wherein the medical device is configured to at least one of store, generate and process data corresponding to at least one of a date the medical device is operated, a time the medical device is operated, a length of time that the medical device is operated, a medical facility department that used the medical device, a medical facility room in which the medical device is used, a serial number of the medical device and a usage of the medical device.

14. The system according to any of the preceding claims, wherein the information system includes a medical facility information network connected to a medical facility server, and wherein the medical facility server is configured to process at least one of patient data, prescription data and inventory/ordering data.

15. The system according to any of the preceding claims, wherein the medical device is one of a surgical stapler, a surgical cutter, a surgical stapler-cutter, a linear surgical stapler, a linear surgical stapler-cutter, a circular surgical stapler, a circular surgical stapler-cutter, a surgical clip applier, a surgical clip ligator, a surgical clamping device, a vessel expanding device, a lumen expanding device, a scalpel and a fluid delivery device.
